# EUROPEAN PATENT APPLICATION

(11) **EP 4 717 227 A1**
(43) Date of publication of application: **01.04.2026**
(21) Application number: 24900595.0
(22) Date of filing: 03.12.2024
(51) Int. Cl.: A61C 7/08, A61C 19/06, A61N 5/06

(54) **INTRAORAL LIGHT IRRADIATION DEVICE**

(30) Priority: 06.12.2023 JP 2023206237
(71) Applicant: Japan PBM Healing Kabushiki Kaisha, Tokyo, 102-0084 (JP); Japan PBM Healing Kabushiki Kaisha, Tokyo 108-0023 (JP)
(72) Inventor: KWONG HING Alan, 75004 Paris (FR)
(74) Representative: Gislon, Gabriele
(86) International application number: PCT/JP2024/042643
(87) International publication number: WO 2025/121301

(57) **Abstract**

An object of the present invention is to provide an intraoral light irradiation device that has excellent wearing comfort and accurately irradiates light to the attached gingiva and alveolar mucosa of orthodontic teeth.

An intraoral light irradiation device 1 includes a mouthpiece 2 formed in a U-shape, and a light source 4 disposed in the mouthpiece 2 and provided with a plurality of light emitters 3 irradiating light to the wearer's gums. The mouthpiece 2 includes a bite tray 5 formed in a U-shape, a front flange 6 facing outer surfaces of the wearer's gums, and a rear flange 7 facing inner surfaces of the wearer's gums. The front and rear flanges 6, 7 are optically transparent, and the light source 4 is disposed to be capable of irradiating light from an inner peripheral surface of the front flange 6 and an outer peripheral surface of the rear flange 7. Each end portion of the front flange 6 is formed with a protruding portion 11 protruding in an extending direction of the front flange 6 from the bite tray 5.

## Description

### FIELD OF THE INVENTION

The present technique relates to an intraoral light irradiation device used in orthodontics.

### BACKGROUND OF THE INVENTION

Orthodontics is performed by elastically compressing orthodontic teeth that need correction. There is an alveolar bone in the gums supporting the teeth, and a periodontal ligament between the alveolar bone and the root of the teeth. The periodontal ligament is a cushioning membrane that provides a cushioning effect to absorb the impact forces acting on the teeth. When the orthodontic teeth are elastically pressed in a particular direction, the periodontal ligament on the pressed side is compressed, and the periodontal ligament on the opposite side is stretched. When the periodontal ligament is compressed, stress is generated in the alveolar bone, which serves as an inflammatory stimulus. This triggers the release of inflammatory stimulants that induce the appearance and activation of osteoclasts, dissolving the alveolar bone and leading to bone resorption. When the periodontal ligament is stretched, teeth-forming osteoblasts appear and newly form the alveolar bone. When the orthodontic teeth are continuously and elastically compressed, the teeth gradually move in the direction of compression by repeating dissolution and formation of the alveolar bone.

In conventional orthodontics, an orthodontic appliance is attached to the crown portions of the orthodontic teeth, which applies elastic pressure, but there is a possibility that a large amount of force is temporarily applied by the orthodontic appliance. In addition, the orthodontic appliance is more likely to cause dental caries and stomatitis, and directly applies a force to the orthodontic teeth, which may damage the roots of the teeth, causing long-term stress on the teeth and periodontal tissues.

Therefore, as a method to increase the speed of tooth movement without damaging teeth and periodontal tissues, a method to improve bone resorption and regeneration by light irradiation has been proposed. Light irradiation is known to be effective in the treatment of bone disease and the biostimulation of bone and soft tissue, and can effectively contribute to the acceleration of resorption and regeneration of alveolar bone.

### PRIOR ART

### PATENT DOCUMENTS

Patent Document 1: JP 2016-538028 A

### SUMMARY OF THE INVENTION

### PROBLEMS TO BE SOLVED BY THE INVENTION

As a light irradiation device for orthodontics, an intraoral light irradiation device of mouthpiece type is known (see Patent Document 1). However, such an intraoral light irradiation device of mouthpiece type is required to improve wearing comfort, for example, reducing a vomiting reflex when the mouthpiece is fit into a user's oral cavity. Particularly, for orthodontics in molars and wisdom teeth, a shape that accounts for wearing comfort when the mouthpiece is fit to the deep interior of the oral cavity is required.

Among the gums of orthodontic teeth, it is necessary to accurately irradiate the attached gingiva and its upper or lower alveolar mucosa. Irrespective of the position of orthodontic teeth, such as particularly molars, wisdom teeth, or front teeth, it is required to accurately irradiate the attached gingiva and its upper or lower alveolar mucosa.

In view of the above disadvantages, an object of the present technique is to provide an intraoral light irradiation device of mouthpiece type that has excellent wearing comfort, and accurately irradiates light to the attached gingiva and alveolar mucosa of orthodontic teeth.

### MEANS FOR SOLVING THE PROBLEMS

In order to solve the above problems, an intraoral light irradiation device according to the present technique includes a mouthpiece that is formed in a U-shape according to a wearer's dentition and is fit in an oral cavity, and a light source that is disposed in the mouthpiece and is provided with a plurality of light emitters, such that the intraoral light therapy device irradiates light to the wearer's gums from the light emitters, wherein the mouthpiece includes:
a bite tray formed in a U-shape according to the wearer's dentition;
a front flange formed vertically along one side edge of the bite tray and facing outer
surfaces of the wearer's upper and lower gums; and
a rear flange formed vertically along the other side edge of the bite tray and facing inner
surfaces of the wearer's upper and lower gums; and

wherein the front and rear flanges are optically transparent, and the light source is disposed to be capable of irradiating light from an inner peripheral surface of the front flange and an outer peripheral surface of the rear flange, and
wherein each end portion of the front flange is formed with a protruding portion protruding in an extending direction of the front flange from the bite tray.

Moreover, another intraoral light irradiation device according to the present technique includes a mouthpiece that is formed in a U-shape according to a wearer's dentition and is fit in an oral cavity, and a light source that is disposed in the mouthpiece and is provided with a plurality of light emitters, such that the intraoral light therapy device irradiates light to the wearer's gums from the light emitters, wherein the mouthpiece includes:
a bite tray formed in a U-shape according to the wearer's dentition;
a front flange formed vertically along one side edge of the bite tray and facing outer surfaces of the wearer's upper and lower gums; and
a rear flange formed vertically along the other side edge of the bite tray and facing inner surfaces of the wearer's upper and lower gums; and

wherein the front and rear flanges are optically transparent, and the light source is disposed to be capable of irradiating light from an inner peripheral surface of the front flange and an outer peripheral surface of the rear flange, and
wherein the front flange is formed with a slit from an upper surface downward, such that the upper labial frenulum or lower labial frenulum of the wearer is located at the slit.

### ADVANTAGES OF INVENTION

According to the present technique, it is possible to provide an intraoral light irradiation device that has excellent wearing comfort and accurately irradiates light to the gums and alveolar mucosa of orthodontic teeth.

### BRIEF DESCRIPTION OF THE DRAWINGS

Fig. 1 is a top plan view showing an intraoral light irradiation device to which the present technique is applied.
Fig. 2 is a front elevational view showing the intraoral light irradiation device.
Fig. 3 is a left-side elevational view showing the intraoral light irradiation device.
Fig. 4 is a bottom plan view showing the intraoral light irradiation device.
Fig. 5 is a right-side elevational view showing the intraoral light irradiation device.
Fig. 6 is a rear elevational view showing the intraoral light irradiation device.
Fig. 7A is a perspective view showing the intraoral light irradiation device, in which the light source is not extended to the protruding portions.
Fig. 7B is a perspective view showing the intraoral light irradiation device, in which the light source is extended to the protruding portions.
Fig. 8 is a cross-sectional view showing the positional relation between the light source and the gums when the intraoral light irradiation device is fit.
Fig. 9 is a perspective view showing a variant example of the intraoral light irradiation device.
Fig. 10 is a perspective view showing the intraoral light irradiation device from the upper back.
Fig. 11 is a perspective view showing a state where a mouthpiece is connected to a controller by an electric cable, and showing a power supply unit connected to the controller by another electric cable.
Fig. 12 is a function block diagram showing a configuration example of the controller.
Fig. 13 is a function block diagram showing a configuration example of the intraoral light irradiation device according to an embodiment of the present invention.
Fig. 14 is a drawing showing a using state where the intraoral light irradiation device is fit to the upper dentition.
Fig. 15 is another drawing showing a using state where the intraoral light irradiation device is fit to the upper dentition.

### EMBODIMENTS OF THE INVENTION

An intraoral light irradiation device to which the present technique is applied is explained in detail by referring to the drawings. Incidentally, the present technique is not restricted to the following embodiments, and various modifications may be made within the scope of the gist of the present technique. The drawings are schematic, and the proportions of the dimensions may differ from those of the actual product. Specific dimensions should be determined by taking into consideration the following explanation. Furthermore, there are, of course, differences in the dimensional relations and ratios between the drawings.

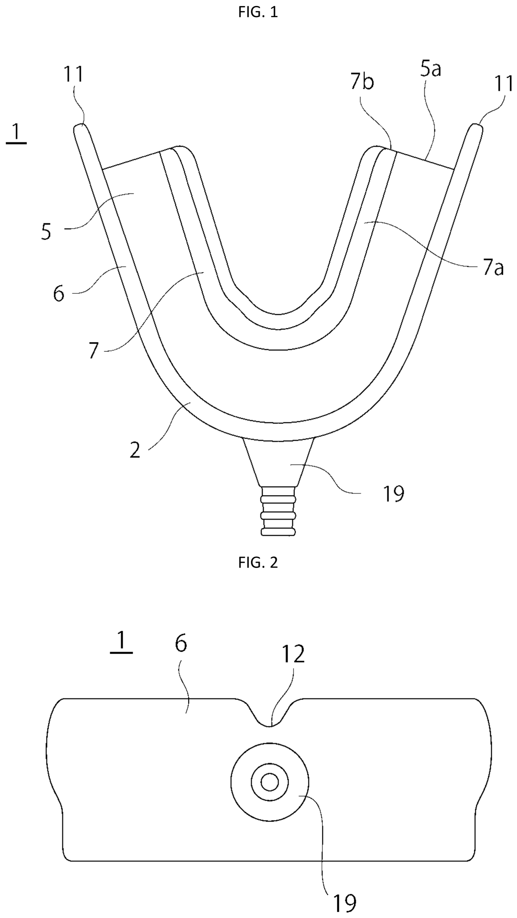

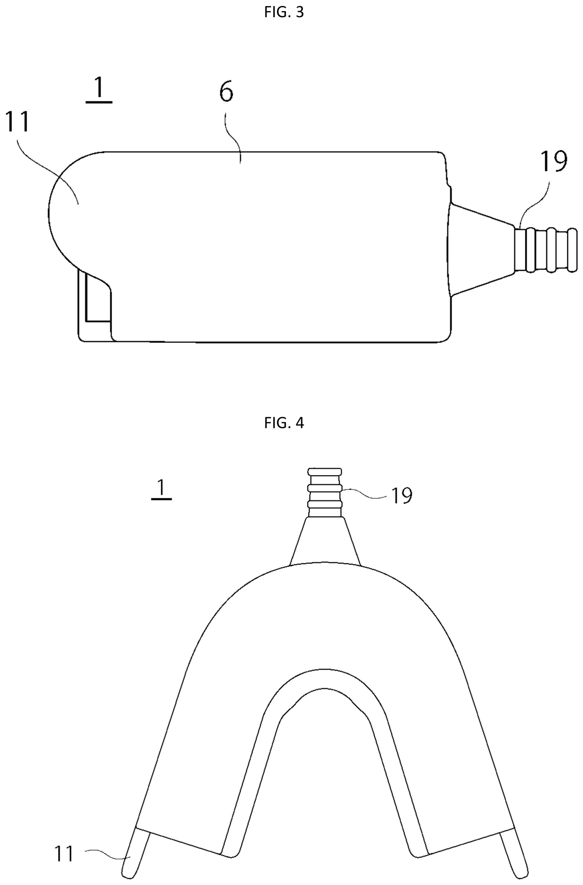

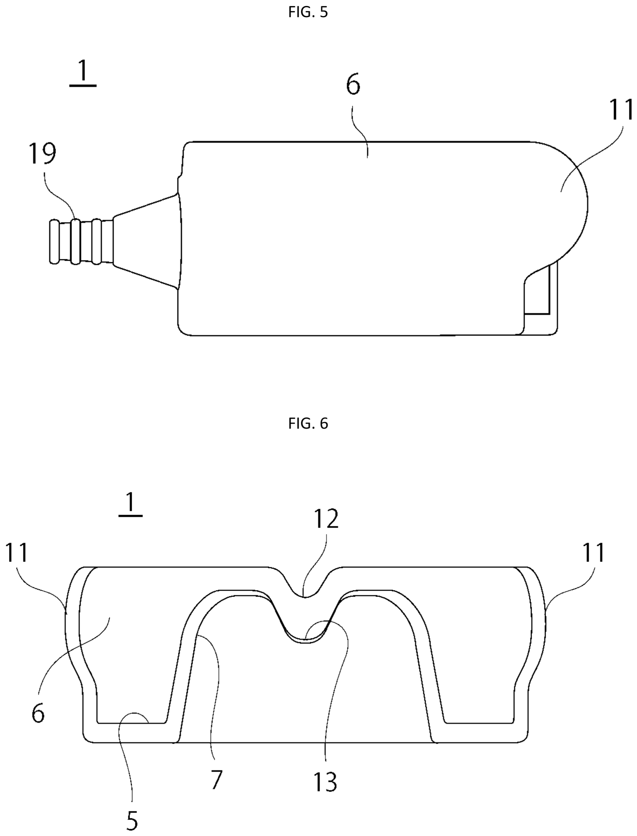

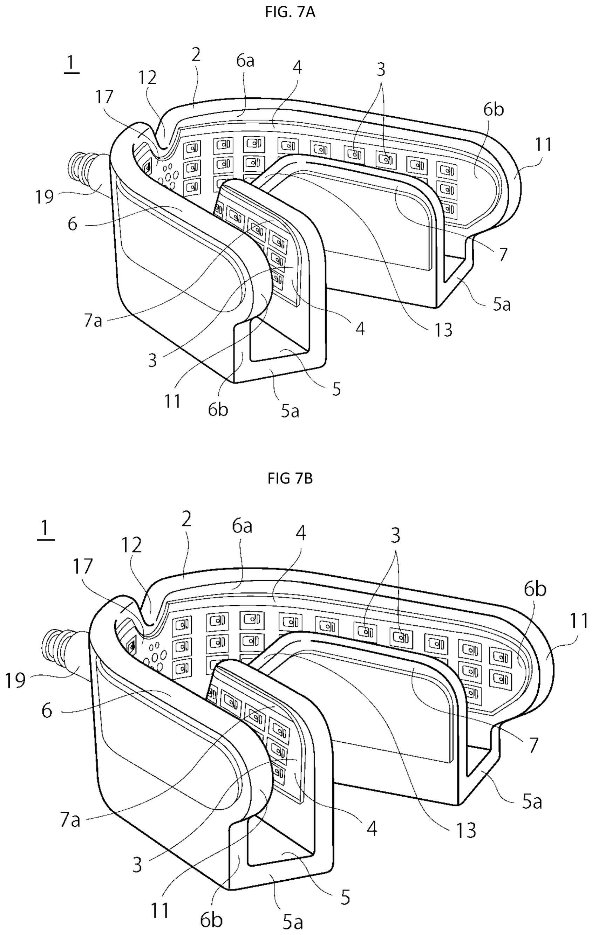

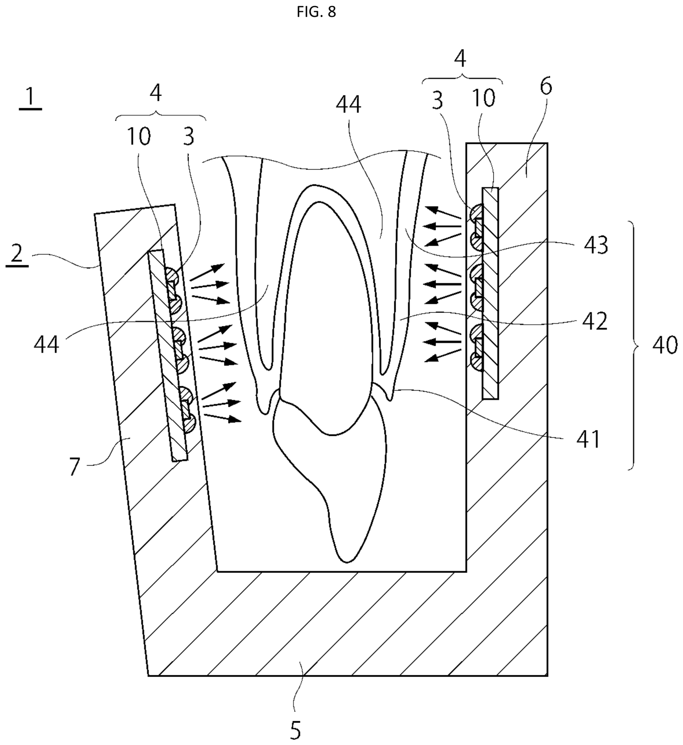

Fig. 1 is a top plan view showing an intraoral light irradiation device to which the present technique is applied. Fig. 2 is a front elevational view showing the intraoral light irradiation device. Fig. 3 is a left-side elevational view showing the intraoral light irradiation device. Fig. 4 is a bottom plan view showing the intraoral light irradiation device. Fig. 5 is a right-side elevational view showing the intraoral light irradiation device. Fig. 6 is a rear elevational view showing the intraoral light irradiation device. Fig. 7 is a perspective view showing the intraoral light irradiation device 1, (A) showing a configuration in which the light source is not extended to the protruding portions, and (B) showing another configuration in which the light source is extended to the protruding portions. Fig. 8 is a cross-sectional view showing the positional relation between the light source and the gums when the intraoral light irradiation device is fit.

The intraoral light irradiation device 1 has a mouthpiece 2 that is fit in an oral cavity and is formed in a U-shape according to a wearer's dentition, and a light source 4 which is disposed in the mouthpiece 2 and in which a plurality of light emitters 3 is arranged.

The mouthpiece 2 includes a bite tray 5 formed in a U-shape according to the wearer's dentition, a front flange 6 formed vertically along one side edge of the bite tray 5 and facing outer surfaces of the wearer's gums, and a rear flange 7 formed vertically along the other side edge of the bite tray 5 and facing inner surfaces of the wearer's gums. Incidentally, in the present specification, the outer surfaces of the gums refer to the surfaces facing the cheeks and lips, and the inner surfaces of the gums refer to the surfaces opposite the outer surfaces of the gums, facing the tongue.

At least an inner peripheral surface 6a of the front flange 6 and an outer peripheral surface 7a of the rear flange 7 of the mouthpiece 2 are optically transparent. The light emitters 3 are arranged in the light source 4 such that light is irradiated from the inner peripheral surface 6a of the front flange 6 and the outer peripheral surface 7a of the rear flange 7. Thus, it is made to be capable of irradiating the light from the light emitters 3 to the outer surfaces and inner surfaces of the wearer's gums. Incidentally, in the present specification, each of the outer peripheral surfaces of the front and rear flanges 6, 7 refers to the surface fronting on the outer side of the oral cavity and facing the cheeks and lips, and each of the inner peripheral surfaces of the front and rear flanges 6, 7 refers to the surface fronting on the inner side of the oral cavity, opposite the paired outer peripheral surface, and facing the tongue.

Fig. 8 is a cross-sectional view showing the positional relation between the light source and the gums when the intraoral light irradiation device 1 is fit into the upper dentition. When the intraoral light irradiation device 1 is fit along the upper dentition or the lower dentition, the light emitters 3 are made to face the attached gingiva and the alveolar mucosa of orthodontic teeth to irradiate the attached gingiva and the alveolar mucosa with a predetermined light. In this drawing, 40 indicates the gums, 41 indicates the free gingiva, 42 indicates the attached gingiva, 43 indicates the alveolar mucosa, and 44 indicates the alveolar bone.

End portions 6b of the front flange 6 are respectively formed with a protruding portion 11 that protrudes from each end portion 5a of the bite tray 5. When the mouthpiece 2 is fit in the oral cavity, each of the protruding portions 11 is inserted into the deep interior of the oral cavity and is placed on the side of the cheek with respect to the retromolar area. Thus, the mouthpiece 2 is fit into the oral cavity snugly and can be held stably with a light pressure without the need to be bitten down hard. Each of the end portions 6b of the front flange 6 fit between the inside of the cheek and the dentition protrudes, and each of the end portions 7b of the rear flange 7 fit between the dentition and the root of the tongue side does not protrude as much as the front flange 6, so that a vomiting reflex is reduced and a wearing comfort is not damaged.

The configuration of the intraoral light irradiation device 1 is described in detail below.

### [Mouthpiece]

The mouthpiece 2 is used so as to cover the upper dentition or the lower dentition of the wearer, and includes the front flange 6 formed vertically along one side edge on the outer peripheral side of the bite tray 5, and the rear flange 7 formed vertically along the other side edge on the inner peripheral side of the bite tray 5. Thus, when the mouthpiece 2 is fit in the oral cavity, the dentition is inserted between the front flange 6 and the rear flange 7. The mouthpiece 2 can be used to cover either the upper dentition or the lower dentition.

The bite tray 5 is a part where the wearer can bite when the wearer holds the mouthpiece 2 in the oral cavity, and is formed in a rough U-shape in a plan view according to the wearer's dentition shape.

When the mouthpiece 2 is fit in the oral cavity, the front flange 6 is opposite to the outer surfaces of the gums, particularly the attached gingiva and its upper or lower alveolar mucosa. The front flange 6 is optically transparent, is provided with the light source 4, and is made to be capable of irradiating the light to the attached gingiva and its upper or lower alveolar mucosa of the outer surfaces of the gums, from the light emitters 3 of the light source 4.

When the mouthpiece 2 is fit in the oral cavity, the rear flange 7 is opposite to the inner surfaces of the gums, particularly the attached gingiva and its upper or lower alveolar mucosa. The rear flange 7 is optically transparent, is provided with the light source 4, and is made to be capable of irradiating the light to the attached gingiva and its upper or lower alveolar mucosa of the inner surfaces of gums, from the light emitters 3 of the light source 4.

Incidentally, the bite tray 5, the front flange 6, and the rear flange 7 maintain their respective shapes while exhibiting moderate flexibility, and are made fittable by following the shape of the inside of the oral cavity.

### [Protruding portion]

Both the end portions 6b of the front flange 6 are respectively formed with a protruding portion 11 that protrudes in an extending direction of the front flange 6 from each of the end portions 5a of the bite tray 5. When the mouthpiece 2 is fit in the oral cavity, each of the protruding portions 11 is placed on the side of the cheek with respect to the retromolar area, is formed in an arc shape in a side view, and each tip surface of them is formed in an arc shape in a plan view, too. Thus, even when the protruding portions 11 contact the buccal mucosa, the wearing comfort is not damaged.

When the mouthpiece 2 is fit in the oral cavity, each of the protruding portions 11 is inserted into the deep interior of the oral cavity and is placed on the side of the cheek. Thus, the mouthpiece 2 is fit into the oral cavity snugly and can be held stably with a light pressure without the need to bite down hard.

Each of the protruding portions 11 is formed to protrude from the side of the upper side edge of the front flange 6. Thus, the protruding portions 11 inserted into the deep interior of the oral cavity are adhered to the gums along the shape inside the oral cavity, so that the front flange 6 is fit into the upper portion of the oral cavity, that is, the top of the backside of the upper lip, or to the lower portion of the oral cavity, that is, the bottom of the backside of the lower lip, and the mouthpiece 2 can be held stably. Moreover, the front flange 6 is opposite to the attached gingiva and alveolar mucosa of the gums. That is, the mouthpiece 2 is configured such that the tooth crowns are positioned on the bottom surface side of the bite tray 5, and the attached gingiva and the alveolar mucosa are positioned on the side of the upper side edge of the front flange 6.

As shown in Figs. 3, 5, 6, 7, since each of the protruding portions 11 is protruded continuously from the upper side edge of the front flange 6, the light source 4 can be formed as close as possible to the side of the upper side edge of the front flange 6, and it is possible to accurately irradiate the light to the attached gingiva and the alveolar mucosa. Moreover, each of the protruding portions 11 is not formed over the entire vertical formation direction of the front flange 6, but is protruded in a length from the upper side edge to about two-thirds in the vertical formation direction, and the lower portions of the respective end portions 6b, where the tooth crowns are located, are respectively flush with the end portions 5a of the bite tray 5. Thus, the light source 4 can be disposed at a position such that it accurately irradiates the attached gingiva and the alveolar mucosa, the protruding portion 11 can be suppressed in size, and the wearing comfort can be maintained.

Incidentally, each of the protruding portions 11 may extend from a position slightly below the upper side edge of the front flange 6, provided that the irradiation position of the light source 4 is not impaired. The protruding portions 11 may extend to the lower portion of the front flange 6 as long as the wearing comfort is not significantly impaired.

As shown in Fig. 7 (B), it is preferable that the light emitters 3 of the light source 4 are also provided in the protruding portion 11 of the mouthpiece 2. The front flange 6 and the rear flange 7 are formed so as to face the molars (second molars) and the wisdom teeth (third molars), and are designed so that the light emitters 3 can irradiate light to these parts. However, depending on the wearer, it is assumed that the molars and wisdom teeth may be located deviating from the front flange 6 and the rear flange 7. In this case too, since each of the protruding portions 11 is formed on the side of the upper side edge of the front flange 6, the protruding portions 11 are confronted with the attached gingiva and the alveolar mucosa of the molars and the wisdom teeth. Then, by providing the light emitters 3 in the protruding portions 11, the light can be irradiated onto the upper or lower attached gingiva and the alveolar mucosa of the molars and the wisdom teeth.

The end portions 7b of the rear flange 7 are respectively formed flush with the end portions 5a of the bite tray 5, and there is no protruding portion to protrude in the extending direction. Since the rear flange 7 is fit between the inside of the dentition and the root of the tongue side, it does not protrude as much as the front flange 6, thereby reducing the vomiting reflex and not damaging the wearing comfort. Incidentally, a protruding portion may be formed on both the end portions 7b of the rear flange 7 to such an extent that the wearing comfort is not impaired by reducing the protruding length or protruding area.

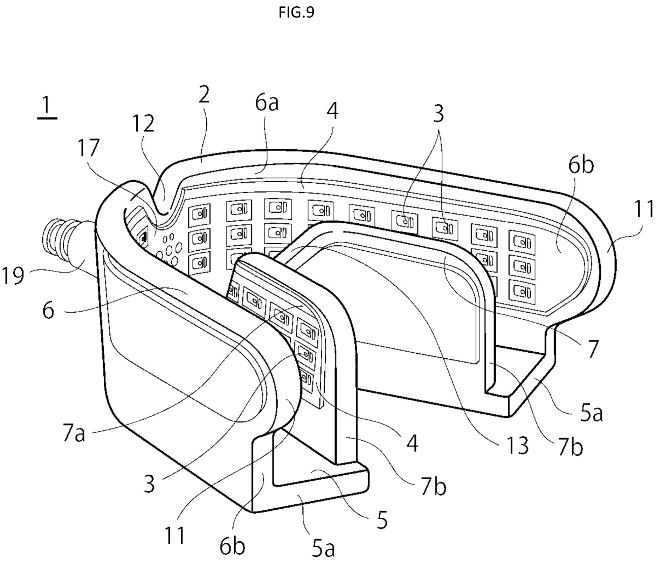

Incidentally, as shown in Fig. 9, the end portions 7b of the rear flange 7 may be shortened so that they are not respectively extended to the end portions 5a of the bite tray 5. By respectively setting back the end portions 7b of the rear flange 7 relative to the end portions 5a of the bite tray 5, the vomiting reflex can be reduced. In this case, it is preferable to form the protruding portions 11 on the front flange 6, but when the main purpose is to reduce the vomiting reflex, the end portions 6b of the front flange 6 may be respectively formed flush with the end portions 5a of the bite tray 5 without providing the protruding portion 11.

### [Slit]

A front slit 12 is formed from the upper side edge downward (the bite tray 5 side) on a central portion of the front flange 6 facing the center of dentition. When the mouthpiece 2 is fit into the oral cavity, an upper labial frenulum (superior labial frenulum) or a lower labial frenulum (inferior labial frenulum) is inserted into the front slit 12. Thus, the front flange 6 can be fit to the upper or lower portion of the oral cavity facing the alveolar mucosa without interference between the front flange 6 and the upper or lower labial frenulum.

On the respective backsides of the upper and lower lips, near the center of dentition, the upper labial frenulum and the lower labial frenulum extend from the alveolar mucosa to the attached gingiva, respectively. Thus, when the front slit 12 is not formed on the central portion of the front flange 6 facing the center of dentition, the upper side edge of the front flange 6 interferes with the upper labial frenulum or the lower labial frenulum, and therefore it is not possible to fit the front flange 6 to the upper portion of the oral cavity, that is, the top of the backside of the upper lip, or to the lower portion of the oral cavity, that is, the bottom of the backside of the lower lip. Moreover, pain or discomfort occurs owing to the interference between the upper side edge of the front flange 6 and the upper labial frenulum or the lower labial frenulum so that the height of the front flange 6 cannot be sufficiently secured, and it makes it difficult to sufficiently face the front flange 6 to the gingival margins or the free gingiva 9b of the vicinity thereof.

Accordingly, since the front flange 6 is formed with the front slit 12, it is possible to fit the front flange 6 to the top of the backside of the upper lip or the bottom of the backside of the lower lip so as to face the front flange 6 to the alveolar mucosa without the interference between the upper side edge of the front flange 6 and the upper labial frenulum or the lower labial frenulum.

A rear slit 13 is formed from the upper side edge downward (the bite tray 5 side) on a central portion of the rear flange 7 facing the center of dentition. When the mouthpiece 2 is fit into the oral cavity, a lingual frenulum (frenulum linguae) or a median palatine raphe (palatine raphe) is inserted into the rear slit 13. Thus, the rear flange 7 can be fit to the upper or lower portion of the oral cavity facing the alveolar mucosa without interference between the rear flange 7 and the lingual frenulum or the median palatine raphe.

The lingual frenulum extends below the tongue near the center of dentition, and the median palatine raphe extends on the palate near the center of dentition. Thus, when the rear slit 13 is not formed on the central portion of the rear flange 7 facing the center of dentition, the upper side edge of the rear flange 7 interferes with the lingual frenulum or the median palatine raphe, and therefore it is not possible to fit the rear flange 7 to the upper portion of the oral cavity, that is, the top of the palate, or to the lower portion of the oral cavity, that is, the bottom of the tongue. Moreover, pain or discomfort occurs owing to the interference between the upper side edge of the rear flange 7 and the lingual frenulum or the median palatine raphe so that the height of the rear flange 7 cannot be sufficiently secured, and it makes it difficult to sufficiently face the rear flange 7 to the attached gingiva or the alveolar mucosa.

Accordingly, since the rear flange 7 is formed with the rear slit 13, it is possible to fit the rear flange 7 to the top of the palate or the bottom of the tongue so as to face the rear flange 7 to the alveolar mucosa without the interference between the upper side edge of the rear flange 7 and the lingual frenulum or the median palatine raphe.

### [Material]

The material of the mouthpiece 2 is the same as that of a mouthpiece conventionally used in the field of sports and medicine, and is formed of a transparent or translucent resin material capable of irradiating light from the light source 4 and having flexibility. Specifically, the material for the mouthpiece 2 can be a resin that conforms to the shape of the oral cavity and has appropriate hardness and softness, such as olefin-based resins, polyester-based resins, urethane-based resins like polyurethane, polyimide-based resins, silicone-based resins, styrene-based resins, acrylic-based resins, polyamide-based resins, and carbonate-based resins. However, it is not limited to these materials.

As the above-described olefin-based resins, the following are preferably exemplified: polyethylene (PE), polyethylene-based resins, polypropylene (PP), polypropylene-based resins, ethylene-vinyl acetate copolymer (EVA), etc., and the following are more preferably exemplified: polyethylene (PE), polyethylene-based resins, polypropylene (PP), polypropylene-based resins, etc.

Polyester-based resins are polycondensates of polycarboxylic acids (such as dicarboxylic acids) and polyalcohol (such as diols). As the above-described polyester-based resins, polyethylene terephthalate (PET) is exemplified.

Urethane-based resins are polycondensates formed from a compound containing isocyanate groups and a compound containing hydroxyl groups. As the above-described urethane-based resins, thermoplastic polyurethane (TPU) is exemplified.

Polyamide-based resins are (co)polymers formed by the bonding of numerous monomers via amide bonds. As the above-described polyamide-based resins, there are examples, such as nylon, para-type amides, and meta-type amides.

Acrylic rubber-based resins are (co)polymers containing acrylic-based rubbers as main components. As the above-described acrylic-based resins, there are examples, such as a block copolymer of methyl methacrylate and butyl acrylate.

Incidentally, the bite tray 5 and the front and rear flanges 6, 7 may be formed from different materials to vary their hardness.

The above-described mouthpiece 2 can be obtained by molding the above-described material integrally with the light source 4. An injection condition for the material is set according to the size and shape of the mouthpiece 2, and typically, the entire mouthpiece 2 is molded in a single mold. The size of the mouthpiece 2 may be suitable for conforming to the dentition of an average person. The mouthpiece 2 of the present invention is manufactured as an adult mouthpiece with a size and shape corresponding to the dentition of an average adult when the wearer is an adult, and as a child mouthpiece with a size and shape corresponding to the dentition of an average child when the wearer is a child.

### [Light source]

The light source 4 disposed in the mouthpiece 2 is explained. The light source 4 includes the substrate 10 and the plurality of light emitters 3 arranged on the substrate 10. The intraoral light irradiation device 1 irradiates the light onto the attached gingiva and the alveolar mucosa by the light emitters 3, and gives a stimulus to the alveolar bone, to cause an inflammatory stimulus on the alveolar bone and activate osteoclasts on the surface of the alveolar bone, thereby causing bone resorption in the alveolar bone. In addition, the light irradiation can suppress bleeding and alleviate discomfort and pain.

As the substrate 10, there are no particular restrictions as long as it is a flexible substrate having flexibility and capable of mounting the light emitters 3. For example, a known flexible substrate using polyimide (PI), liquid crystal polymer (LCP), or the like as a base film can be used. The respective substrates 10 are disposed along the side surfaces of the front and rear flanges 6, 7 of the mouthpiece 2, are formed into an approximately rectangular plate shape according to the shape of the front and rear flanges 6, 7, and are bent into a U-shape to be disposed. The substrates 10 are respectively disposed to be extended to the end portions of the front and rear flanges 6, 7. The substrate 10 is preferably disposed to extend to the protruding portions 11.

The light emitters 3 are arranged on the respective substrates 10 such that the light is emitted from the inner peripheral surface of the front flange 6 and the outer peripheral surface of the rear flange 7. Thus, the light sources 4 are positioned to face each other between the front and rear flanges 6, 7, enabling light to be irradiated onto both surfaces of the attached gingiva and the alveolar mucosa of the orthodontic teeth.

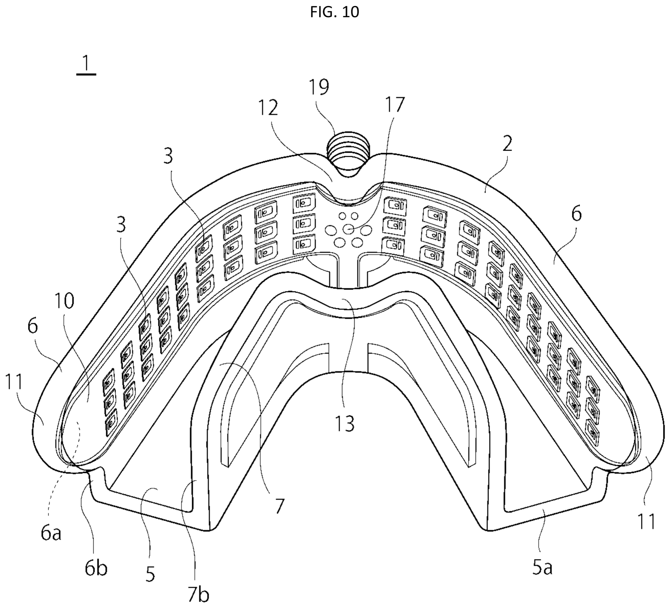

Fig. 10 is a perspective view showing the intraoral light irradiation device 1 from the upper back. As shown in Fig. 10, the substrate 10 disposed in the front flange 6 has a connecting terminal part 17 formed in a central portion facing the center of dentition. Each of the light emitters 3 on the substrate 10 disposed in the front flange 6 is connected to the connecting terminal part 17 via an internal wiring. Each of the light emitters 3 on the substrate 10 disposed in the rear flange 7 is connected to the connecting terminal part 17 via another internal wiring passing through the bite tray 5 at the central portion facing the center of dentition. The connecting terminal part 17 is connected to a controller 16 via an electric cable 15 led out from the outside surface of the central portion of the front flange 6 of the mouthpiece 2. The light source 4 is controlled by the controller 16 connected to the end portion of the electric cable 15 to regulate light irradiation by the light emitters 3 (irradiation ON and OFF, irradiation area, irradiation mode (duration, radiation intensity, irradiation dose, etc.)).

Incidentally, as shown in Fig. 2, a lead-out part 19 where the electric cable 15 is led out is provided slightly below the upper side edge of the outside surface of the front flange 6. This is because, as described above, the front slit 12 is provided in the central portion of the front flange 6, and the electric cable 15 is derived from a portion below the lower end of the front slit 12.

### [Light emitters]

The light emitters 3 are not particularly restricted. As the light emitters 3, there are examples, such as light-emitting diode (LED), organic light-emitting diode (OLED), semiconductor laser diode (LD), polymer light-emitting diode (PLED), light-emitting polymer (LEP), optical fiber bundle, etc., or combinations thereof, but are not limited to these. The light emitters 3 are covered with transparent or translucent resin constituting the mouthpiece 2 by forming the light source 4 integrally with the mouthpiece 2. Thus, the light source 4 is made to be capable of irradiating the light in the oral cavity, and is protected by the resin material when the mouthpiece 2 is used or cleaned.

The plurality of light emitters 3 is mounted along the longitudinal direction of the substrate 10, extending from one end to the other. For example, the light emitters 3 are arranged in 3 columns of 18 to 20 pieces in a grid. Incidentally, the arrangement pattern and number of the light emitters 3 are not limited to these examples. Each of the light emitters 3 may be placed close to an adjacent light emitter 3 so that no areas remain unlit.

As described above, the light emitters 3 are preferably formed in the protruding portions 11 of the front flange 6, each of which is placed on the side of the cheek with respect to the retromolar area, too. Thus, even when the molars and wisdom teeth are located deviating from the front flange 6 and the rear flange 7, the light can be reliably irradiated onto the upper or lower attached gingiva and the alveolar mucosa of the molars and the wisdom teeth.

For the part to be subjected to light irradiation, depending on the settings, the entire light source 4 of the front flange 6 and/or the entire light source 4 of the rear flange 7 may be selected to be irradiated. Further, the irradiation area of each of the light sources of the front and rear flanges 6, 7 may be selected as the whole of the light emitters 3, half of the front teeth side, the other half of the molar side, and so forth. An irradiation pattern may be properly set. The light emitters 3 may be uniformly lit at the same radiation illuminance, or flashed. Moreover, the irradiation position, the radiation intensity, and the irradiation pattern may be set to vary with time.

### [Thermistor]

Incidentally, the substrate 10 may include a temperature detection part 30, such as a thermistor, for detecting the temperature of the light source 4. This allows the light emitters 3 to be controlled to stop the light irradiation when they are heated to a predetermined temperature or higher, thereby protecting the wearer and the mouthpiece 2.

### [Controller]

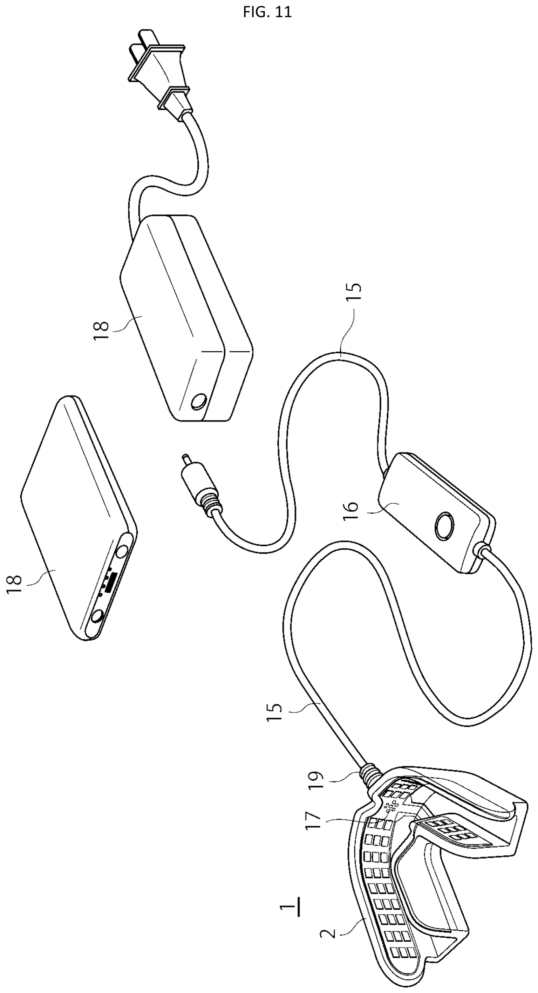

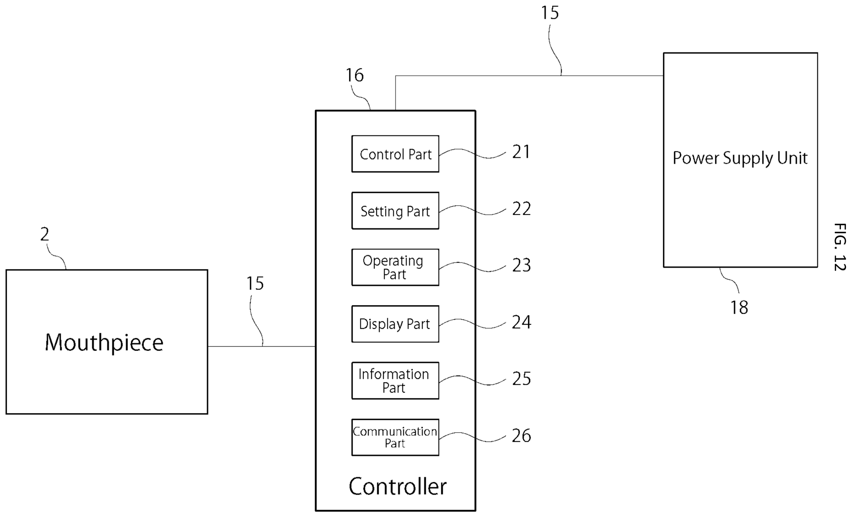

Fig. 11 is a perspective view showing a state where the mouthpiece 2 is connected to the controller 16 by the electric cable 15, and showing a power supply unit 18 connected to the controller 16 by another electric cable 15. The controller 16 is detachably connected to the mouthpiece 2 via the electric cable 15. Fig. 12 is a function block diagram showing a configuration example of the controller 16. As shown in Fig. 12, the controller 16 has a control part 21 and an operating part 23, wherein the control part 21 controls the irradiation area, irradiation time, irradiation wavelength, radiation intensity, etc. of the light emitters 3 based on a program, and stores a mode in which the irradiation area, irradiation time, wavelength of irradiation light, radiation intensity, radiation energy, etc. are set in advance, and the operation history of the intraoral light irradiation device 1 in memory and storage, and wherein the operating part 23 is equipped with a control button for operation such as turning a power source ON/OFF, turning irradiation ON/OFF, and selecting modes.

The controller 16 is further detachably connected to the power supply unit 18 via the power cable. The power supply unit 18 can be a power source device (adapter) connected to a household outlet or a mobile battery containing a rechargeable or disposable battery. In this way, by separating the mouthpiece 2 from the controller 16 and the power supply unit 18, and by connecting them with cables, the weight required when fitting the mouthpiece 2 is only the weight of the mouthpiece 2 itself, and misalignment of a fitting position can be prevented, the wearer's burden of holding the mouthpiece 2 in the oral cavity can be reduced, and further improving operability. Furthermore, since the mouthpiece 2 is detachable from the power cable 15, it is easy to clean only the mouthpiece 2 after use.

Incidentally, the secondary battery may be built into the controller 16 or the mouthpiece 2. The secondary battery built into the controller 16 or the mouthpiece 2 can be charged when the controller 16 or the mouthpiece 2 is connected to the power source device connected to the household outlet, via the power source cable. Additionally, the secondary battery built into the mouthpiece 2 may be rechargeable when the mouthpiece 2 is stored in a mouthpiece case (not shown). In this case, the mouthpiece case is connected to the power source device (adapter) connected to the household outlet, via the power source cable, or the mouthpiece case incorporates the power source device and is connected to the household outlet via the power source cable.

The controller 16 may be provided with a setting part 22 for manually setting the irradiation area, irradiation time, irradiation wavelength, radiation intensity, irradiation energy, etc., a display part 24, such as an LCD panel, that displays the operation condition, operation history, schedule management, remaining light irradiation time, battery level, etc. of the intraoral light irradiation device, an information part 25 that notifies of the completion of a predetermined light irradiation, error states, and so forth, via alarms, light emission, vibration, etc., and a communication part 26 that connects to an external server 31 via the internet and performs renewal of a control program and a schedule stored in the control part 21 and a transmission of the operation history saved in the control part 21.

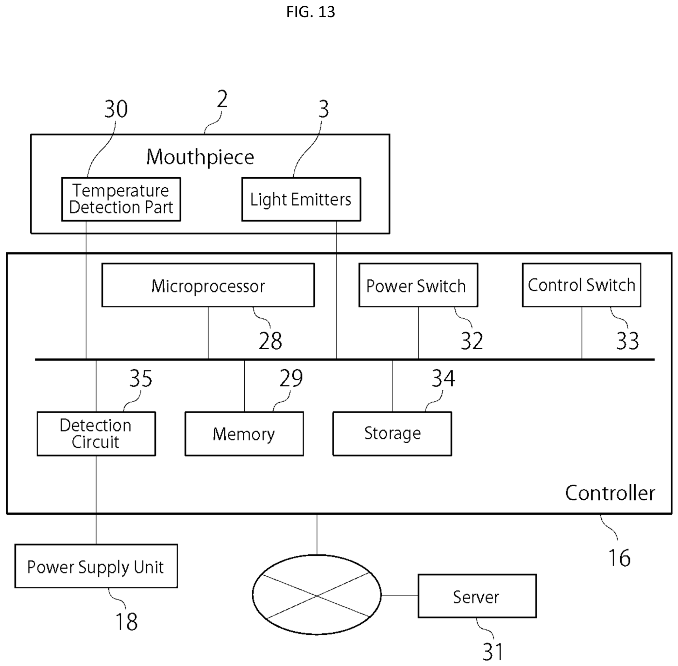

Fig. 13 is a function block diagram showing a configuration example of the intraoral light irradiation device according to an embodiment of the present invention. The control part 21 includes a microprocessor 28. The microprocessor 28 communicates with a memory 29 or a storage 34 about a program such as the irradiation mode, the operation history of the intraoral light irradiation device, and so forth. The microprocessor 28 also saves the setting information of the setting part 22 in the memory 29 or the storage 34, and controls the irradiation according to the setting saved in the memory 29 or the storage 34.

The microprocessor 28 operates according to the operations of a power switch 32, which turns the power source ON/OFF, and a control switch 33, which operates the start/stop of irradiation. The microprocessor 28 also stops the light irradiation in response to a signal from the temperature detection part 30, such as a thermistor. Thus, when the light emitters 3 are heated to a predetermined temperature or more, it is possible to control the intraoral light irradiation device so that the light irradiation is stopped, so as to protect the wearer and the mouthpiece 2.

Furthermore, the microprocessor 28 receives an output of a detection circuit 35 for detecting an output voltage when the power supply unit 18 is configured with a mobile battery, judges a charge amount, and controls the display part 24 and the information part 25 to inform a user.

### [Usage]

The usage of the intraoral light irradiation device is described below. The intraoral light irradiation device is used by a user in orthodontics for a predetermined period, for example, once to several times a day, for a predetermined time per 1 time or per day (in the example described later, about 4 minutes per day for each area requiring correction).

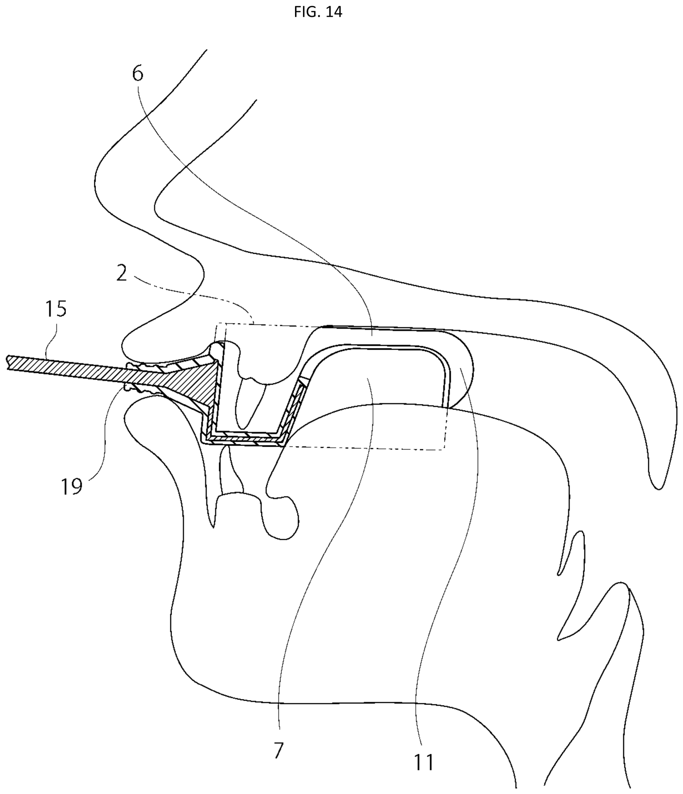

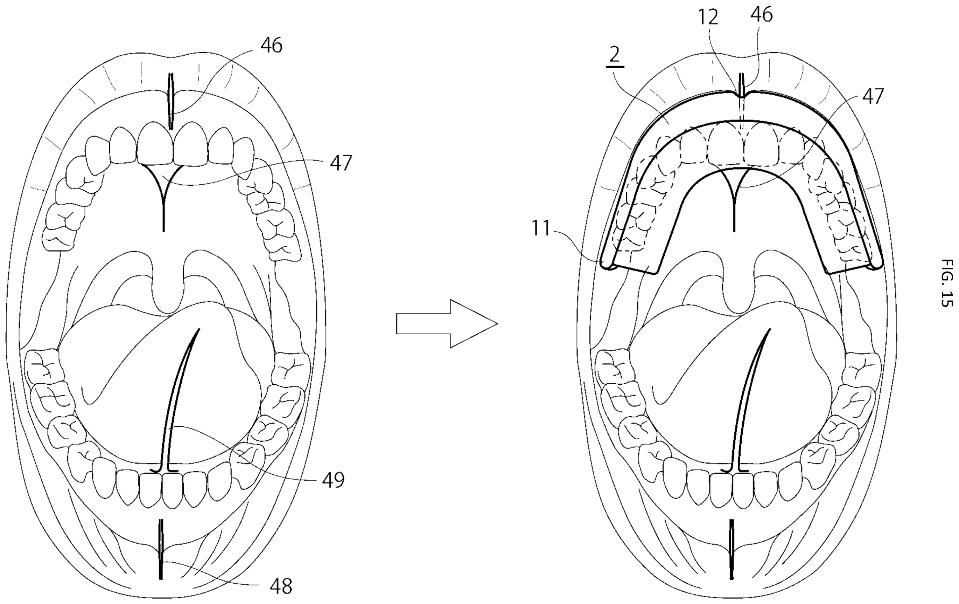

When the intraoral light irradiation device 1 is used for the upper dentition, as shown in Figs. 14, 15, the mouthpiece 2 is fit while facing the U-shaped groove formed by the front flange 6, the rear flange 7, and the bite tray 5 to the upper dentition. At this time, since the front slit 12 is provided on the front flange 6, the mouthpiece 2 can be fit to the top of the backside of the upper lip, without the interference between the front flange 6 and the upper labial frenulum 46. Moreover, since the rear slit 13 is provided on the rear flange 7, the mouthpiece 2 can be fit to the top of the palate, without the interference between the rear flange 7 and the median palatine raphe 47. Thus, the respective light sources 4 provided in the front and rear flanges 6, 7 can be made to face the attached gingiva and the alveolar mucosa.

When the mouthpiece 2 is fit in the oral cavity, each of the protruding portions 11 is inserted into the deep interior of the oral cavity and is placed on the side of the cheek with respect to the retromolar area. Thus, the mouthpiece 2 is snugly fit between the upper dentition gums and the upper lip and cheek, and can be held stably with a light pressure without the need to be bitten down hard.

Incidentally, when the light emitters 3 are arranged in the protruding portions 11, it is possible to reliably irradiate light to the molars of the upper dentition and the attached gingiva and alveolar mucosa of the wisdom teeth, combined with the effects of the front and rear slits 12, 13 described above.

When the intraoral light irradiation device 1 is used for the lower dentition, the mouthpiece 2 is fit while facing the U-shaped groove formed by the front flange 6, the rear flange 7, and the bite tray 5 to the lower dentition. At this time, since the front slit 12 is provided on the front flange 6, the mouthpiece 2 can be fit to the bottom of the backside of the lower lip 48, without the interference between the front flange 6 and the lower labial frenulum 48. Moreover, since the rear slit 13 is provided on the rear flange 7, the mouthpiece 2 can be fit to the bottom of the tongue, without the interference between the rear flange 7 and the lingual frenulum 49. Thus, the respective light sources 4 provided in the front and rear flanges 6, 7 can be made to face the alveolar mucosa.

When the mouthpiece 2 is fit in the oral cavity, each of the protruding portions 11 is inserted into the deep interior of the oral cavity and is placed on the side of the cheek with respect to the retromolar area. Thus, the mouthpiece 2 is snugly fit between the upper dentition gums and the upper lip and cheek, and can be held stably with a light pressure without the need to be bitten down hard.

Incidentally, when the light emitters 3 are arranged in the protruding portions 11, it is possible to reliably irradiate light to the molars of the lower dentition and the attached gingiva and alveolar mucosa of the wisdom teeth, combined with the effects of the front and rear slits 12, 13 described above.

The wavelength of light (Wavelength: nm), the radiation illuminance (Radiation Flux: W/m²), the irradiation area, and the irradiation time are not specifically limited. These parameters are appropriately set so that the stimulus given to the light irradiation area acts as the inflammatory stimulus to activate the osteoclasts, while not damaging the teeth, the gums, the periodontal ligament, the alveolar bone, etc.

Specifically, the wavelength of light (Wavelength: nm) may be set within a range of 850 nm ± 10% (765 to 935 nm), for example. The radiation illuminance (Radiation Flux) of each of the light emitters 3 may be set within a range of 68.64 mW/cm² ± 10% (61.78 to 75.50 mW/cm²), for example. The irradiation area may be possible to be selected from the following modes: irradiating only the light source 4 of the front flange 6 (mode 1), irradiating only the light source 4 of the rear flange 7 (mode 2), and irradiating from the light sources 4 of the front and rear flanges 6, 7 (mode 3). The irradiation time may be set within a range of 240 sec ± 10% (210 to 265 sec), for example. The absorbed energy (Energy Absorb: J) of the light irradiated from the front flange 6 may be set within a range of 889.6 J ± 10% (800.6 to 978.6 J), and the absorbed energy (Energy Absorb: J) of the light irradiated from the rear flange 7 may be set within a range of 428.3 J ± 10% (385.5 to 471.1 J). The operating current can be designed properly. It may be set within a range of 26.0 mA ± 10% (23.4 to 28.6 mA), for example.

For example, when 54 pieces of LEDs are provided as the light emitters 3 in the front flange 6, 26 pieces of LEDs are provided as the light emitters 3 in the rear flange 7, and the radiation illuminance of each light emitters 3 is 68.64 mW/cm², the radiation illuminance from the front flange 6 is apparently 3706.56 mW/cm², and the radiation illuminance from the rear flange 7 is apparently 1784.64 mW/cm². In this setting, when irradiating for 240 seconds, the absorbed energy (Energy Absorb: J) of the light irradiated from the front flange 6 is 889.6 J, and the absorbed energy (Energy Absorb: J) of the light irradiated from the rear flange 7 is 428.3 J. Incidentally, the numerical ranges for the above-described wavelength (nm), radiation illuminance (mW/cm²), and absorbed energy (J) are merely examples, and these values may be set outside the corresponding numerical ranges as long as the effects of the present invention are achieved. As described above, the selection of each mode can be performed using controller 16. The settings of the intraoral light irradiation device 1 are not restricted to those described above.

Incidentally, the radiation illuminance can be obtained by irradiating from the light emitters 3 and measuring the radiant flux of light incident per unit area with a radiation intensity meter. The total radiation illuminance from the front and rear flanges 6, 7 can be calculated by multiplying the radiation illuminance of each of the light emitters and the number of light emitters. The absorbed energy (W·sec) of the light irradiated from the front and rear flanges 6, 7 can be calculated by multiplying the total radiation illuminance (W) and the irradiation time (sec).

Using controller 6, when the operation to start irradiation is performed after selecting the mode, light irradiation is executed according to the selected mode. After the light irradiation is completed, the mouthpiece 2 can be removed from the oral cavity and cleaned.

### REFERENCE SIGNS LIST

- 1: intraoral light irradiation device
- 2: mouthpiece
- 3: light emitters
- 4: light source
- 5: bite tray
- 5a: end portion
- 6: front flange
- 6a: inner peripheral surface
- 6b: end portion
- 7: rear flange
- 7a: outer peripheral surface
- 7b: end portion
- 11: protruding portion
- 12: front slit
- 13: rear slit
- 15: electric cable
- 16: controller
- 17: connecting terminal part
- 18: power supply unit
- 19: lead-out part
- 21: controlpart
- 22: setting part
- 23: operating part
- 24: display part
- 25: information part
- 26: communication part
- 28: microprocessor
- 29: memory
- 30: temperature detection part
- 31: server
- 32: power switch
- 33: control switch
- 34: storage
- 35: detection circuit

## Claims

1. An intraoral light therapy device comprising a mouthpiece that is formed in a U-shape according to a wearer's dentition and is fit in an oral cavity, and a light source that is disposed in the mouthpiece and is provided with a plurality of light emitters, such that the intraoral light therapy device irradiates light to the wearer's gums from the light emitters,
wherein the mouthpiece comprises:
a bite tray formed in a U-shape according to the wearer's dentition;
a front flange formed vertically along one side edge of the bite tray and facing outer surfaces of the wearer's upper and lower gums; and
a rear flange formed vertically along the other side edge of the bite tray and facing inner surfaces of the wearer's upper and lower gums; and
wherein the front and rear flanges are optically transparent, and the light source is disposed to be capable of irradiating light from an inner peripheral surface of the front flange and an outer peripheral surface of the rear flange, and
wherein each end portion of the front flange is formed with a protruding portion protruding in an extending direction of the front flange from the bite tray.

2. The intraoral light therapy device according to claim 1, wherein each end portion of the front flange is formed into an arc shape in a side view.

3. The intraoral light therapy device according to claim 1 or 2, wherein end portions of the rear flange are respectively formed flush with end portions of the bite tray.

4. The intraoral light therapy device according to claim 1 or 2, wherein the light source is extended to the protruding portions.

5. An intraoral light therapy device comprising a mouthpiece that is formed in a U-shape according to a wearer's dentition and is fit in an oral cavity, and a light source that is disposed in the mouthpiece and is provided with a plurality of light emitters, such that the intraoral light therapy device irradiates light to the wearer's gums from the light emitters,
wherein the mouthpiece comprises:
a bite tray formed in a U-shape according to the wearer's dentition;
a front flange formed vertically along one side edge of the bite tray and facing outer surfaces of the wearer's upper and lower gums; and
a rear flange formed vertically along the other side edge of the bite tray and facing inner surfaces of the wearer's upper and lower gums; and
wherein the front and rear flanges are optically transparent, and the light source is disposed to be capable of irradiating light from an inner peripheral surface of the front flange and an outer peripheral surface of the rear flange, and
wherein the front flange is formed with a slit from an upper surface downward, such that the upper labial frenulum or lower labial frenulum of the wearer is located at the slit.

6. The intraoral light therapy device according to claim 5, wherein the rear flange is formed with a slit from an upper surface downward, such that the lingual frenulum of the wearer is located at the slit.

7. The intraoral light therapy device according to claim 1 or 5, wherein a controller capable of controlling the light source is provided, the controller being detachably connected to the mouthpiece by a cable.

8. The intraoral light therapy device according to claim 7, wherein an external power supply unit driving the light source is provided, the external power supply unit being detachably connected to the controller by another cable.

9. The intraoral light therapy device according to claim 7, wherein a power source driving the light source is built into the mouthpiece or the controller.

10. An intraoral light therapy device comprising a mouthpiece that is formed in a U-shape according to a wearer's dentition and is fit in an oral cavity, and a light source that is disposed in the mouthpiece and is provided with a plurality of light emitters, such that the intraoral light therapy device irradiates light to the wearer's gums from the light emitters,
wherein the mouthpiece comprises:
a bite tray formed in a U-shape according to the wearer's dentition;
a front flange formed vertically along one side edge of the bite tray and facing outer surfaces of the wearer's upper and lower gums; and
a rear flange formed vertically along the other side edge of the bite tray and facing inner surfaces of the wearer's upper and lower gums; and
wherein the front and rear flanges are optically transparent, and the light source is disposed to be capable of irradiating light from an inner peripheral surface of the front flange and an outer peripheral surface of the rear flange, and
wherein end portions of the rear flange are respectively set back from end portions of the bite tray.
